# EUROPEAN PATENT APPLICATION

(11) **EP 3 501 540 A1**
(43) Date of publication of application: **26.06.2019**
(21) Application number: 17306858.6
(22) Date of filing: 20.12.2017
(51) Int. Cl.: A61K 45/00, A61K 31/495, A61P 35/00

(54) **MITOCHONDRIAL TRIFUNCTIONAL PROTEIN (MTP) INHIBITORS FOR USE IN THE TREATMENT OF HIGH MITOCHONDRIAL RESPIRATION (OX+) CANCERS**

(71) Applicant: UNIVERSITE DE BORDEAUX, 33000 Bordeaux (FR); INSERM (Institut National de la Santé et de la Recherche Médicale), 75013 Paris (FR)
(72) Inventor: ROSSIGNOL, Rodrigue, 33700 MERIGNAC (FR); DIAS AMOEDO, Nivea, 33700 Mérignac (FR)
(74) Representative: Cabinet Plasseraud

(57) **Abstract**

The present invention describes the identification of two cancer sub-groups based on their bioenergetics status: high mitochondrial respiration (OX+) and low mitochondrial respiration (OX-). Advantageously, it has been discovered that inhibition of the mitochondrial trifunctional protein (MTP) altered high mitochondrial respiration (OX+) tumor growth *in vitro* and *in vivo.*

Hence, the present invention relates to MTP inhibitor for use in the treatment of high mitochondrial respiration (OX+) cancers. It has also been established that high mitochondrial respiration (OX+) cancer cells use an energy source other than glucose such as fatty acids for their growth, survival and resistance to treatments or activation of metastatic processes. These cancers are hence high fatty-acid oxidation dependent (FAO+) cancers. The present invention thus also relates to MTP inhibitor or use in the treatment of high mitochondrial respiration (OX+) cancers and high fatty-acid oxidation (FAO+) cancers. It has also been determined that MTP expression in tumors allows determining the prognosis of a subject suffering from high mitochondrial respiration (OX+) cancers.

## Description

### FIELD OF THE INVENTION

The present invention relates to the field of cancerology, and in particular to the treatment of high mitochondrial respiration (OX+) cancers.

### BACKGROUND OF THE INVENTION

Precision medicine is the basis of novel targeted treatment of many cancers. It is based on the identification of genetic particularities of tumors that reveal a greater sensitivity to a given treatment.

Among the different emerging strategies for cancer therapy, targeting tumor bioenergetics may be promising since rewiring of the biochemical pathways that are involved in energy metabolism contributes to cancer cell growth, survival, epigenetics and metastasis. Metabolic reprogramming is considered as an emergent hallmark of cancer, but the details of the bioenergetic changes and vulnerabilities of human cancer remain uncharacterized.

Two main pathways transduce energy in cancer cells: the anaerobic pathway and the oxidative pathway. The former includes glycolysis that is linked to lactate production and generates two molecules of ATP and two molecules of lactate per molecule of glucose (Warburg effect). The oxidative pathway requires mitochondrial oxygen consumption and generates a higher yield of ATP using a variety of fuels. The oxidative pathway further connects to glycolysis and redirects the end-product pyruvate to the Krebs cycle, rather than to lactate dehydrogenase, thereby abrogating the Warburg effect. Hence, the oxidative pathway can produce 36 ATP molecules per glucose molecule and up to 129 molecules of ATP per molecule of palmitic acid consumed. Therefore, fatty-acid oxidation (FAO) that is linked to oxidative phosphorylation can maximize ATP synthesis, and this catabolic pathway is preferred in a large variety of tumor types.

At present, the metabolic and energetic status of tumors is not evaluated or considered clinically. Metabolic and energetic status can be suggested by the [¹⁸F]-FDG PET Scan examination that relies on the accumulation of labeled non-metabolizable glucose in cancer cells. The commonly accepted idea is that highly proliferating cancer cells consume a lot of glucose and implement molecular strategies to import it (overexpression of GLUT transporter) and then convert it to cellular energy (activation of glycolysis). Thus, the result of the PET Scan examination, would allow a priori to segregate tumors strongly or weakly glucose consumer. However, many studies describe the existence of PET-scan negative tumors, but their biology and bioenergetics remains incomplete and very little studied. A low incorporation of glucose does not exclude the hypothesis that these tumors can use other sources of energy.

Hence, peculiarities in tumor metabolism can eventually be targeted for anti-cancer therapy, but inter-patient and intra-tumor heterogeneity must be considered in the early design of bioenergetic therapeutic approaches. However, limited knowledge regarding the bioenergetics of tumors in vivo and the dramatic metabolic flexibility of cancer cells prevents the development of efficient bioenergetic therapies.

Accordingly, there remains a need for developing efficient bioenergetic medicine.

### SUMMARY OF THE INVENTION

The present invention describes the identification of two cancer sub-groups based on their bioenergetics status: high mitochondrial respiration (OX+) and low mitochondrial respiration (OX-).

Advantageously, it has been discovered that inhibition of the mitochondrial trifunctional protein (MTP) altered high mitochondrial respiration (OX+) tumor growth *in vitro* and *in vivo.*

Hence, the present invention relates to MTP inhibitor for use in the treatment of high mitochondrial respiration (OX+) cancers.

It has also been established that high mitochondrial respiration (OX+) cancer cells use an energy source other than glucose such as fatty acids for their growth, survival and resistance to treatments or activation of metastatic processes. These cancers are hence high fatty-acid oxidation dependent (FAO+) cancers.

The present invention thus also relates to MTP inhibitor or use in the treatment of high mitochondrial respiration (OX+) cancers and high fatty-acid oxidation (FAO+) cancers.

It has also been determined that MTP expression in tumors allows determining the prognosis of a subject suffering from high mitochondrial respiration (OX+) cancers.

### BRIEF DESCRIPTION OF THE DRAWINGS

**FIGURE 1****:** Schematic representation of mitochondrial trifunctional protein and regulation pathway
**FIGURE 2****:** Mitochondrial respiration in intact surgical pieces obtained from nineteen patients with human non-small cell lung cancer (NSCLC) following surgery and dissection by the pathologist in two zones: the proliferating cancer region and the center of the tumor, that was excluded from the analysis because of the necrosis (PZ) and the cancer adjacent tissue, i.e., the external healthy tissue at the distance of the tumor (HT).
   Mitochondrial respiration was measured in tissues using high-resolution respirometry (HRR). The group (N=10) with a high respiratory rate was denoted "OX+", and the group with low respiratory rate was denoted "OX-" (N=9).
**FIGURE 3****:** Ratio between tumor (PZ) and adjacent tissue respiration (HT) enabling a strong (p < 0.0001) segregation of the surgical pieces in two bioenergetics groups based on their respiratory rate, as distinguished by the Anova analysis: high mitochondrial respiration (OX+) and low mitochondrial respiration (OX-).
**FIGURE 4****:** [¹⁸F]-fluorodeoxy-glucose (FDG) standardized uptake values (SUVmax) determined by positron emission tomography (PET) in high mitochondrial respiration (OX+) group and low mitochondrial respiration (OX-) group.
**FIGURE 5****:** Tumor size (in µm) in high mitochondrial respiration (OX+) group and low mitochondrial respiration (OX-) group.
**FIGURE 6****:** Cumulated exposure to tobacco smoke quantified by the number of cigarette pack-years (nPA) in high mitochondrial respiration (OX+) group and low mitochondrial respiration (OX-) group (p=0.0129).
**Figure 7****:** Main proteome differences (label-free proteomics) between OX+PZ and OX+HT determined on six patients stratified using the high-resolution respirometry analysis of PZ and HT. The proteins involved in fatty acid oxidation are shown in grey. The mitochondrial trifunctional protein (MTP) composed of alpha (HADHA) and beta (HADHB) subunits is increased in OX+ tumors.
**FIGURE 8****:** In silico analysis of HADHA expression in the TCGA (The Cancer Genome Atlas) cohort of human lung adenocarcinoma (LUAD) demonstrating a lower survival of the patients with high HADHA tumor expression (HADHA+).
**FIGURE 9****:** HADHA expression level (western blot) in A549 and H460 human lung adenocarcinoma cell lines demonstrating contrasting bioenergetic profiles in good correspondence with high mitochondrial respiration OX+ (A549) and low mitochondrial respiration OX- (H460) in lung adenocarcinoma cells. Expression levels of four enzymes (ETFA, ETFB, ACADVL and Actin) involved in fatty-acid beta oxidation in the mitochondrion were also determined by Western Blot in the two cell lines and significant increased levels are found in the A549 (OX+) cells.

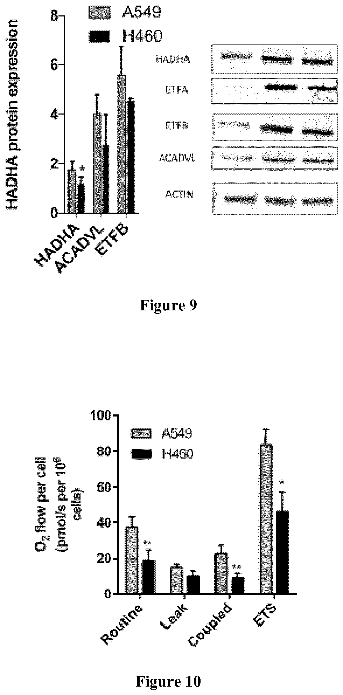

**FIGURE 10****:** Differences in respiratory capacity between A549 and H460 human lung adenocarcinoma cell lines using high-resolution respirometry (HRR). The routine state is the respiration of the cells observed in the culture medium. The leak respiration is obtained after inhibition of the ATP-synthesis machinery and gives a measure of the respiration when mitochondrial energy production is not activated. The coupled respiration is the part of respiration used to produce ATP and is the oligomycin-sensitive (ATP synthase inhibitor) part. The ETS is the maximal (uncoupled) respiration obtained by forcing the respiratory system with a chemical uncoupler. ETS reveals the capacity of the respiratory system.
**FIGURE 11****:** Impact of MTP downregulation using a HADHA-targeting shRNA on cell viability in A549 (OX+) cells (Figures 11A et B) or H460 (OX-) cells (Figure 11 C).
**FIGURE 12****:** Effect of dose-dependent inhibition of MTP on A549 (OX+) cell viability by trimetazidine (TMZ) *in vitro* (Figure 12A) and lack of effect in H460 (OX-) cell viability *in vitro* (Figure 12B).
**FIGURE 13****:** Impact of glucose removal from the medium on A549 (OX+) and H460 (OX-) cell viability.
**FIGURE 14****:** (**A**) Mass spectrometry lipidomic analysis of A549 cells treated with saline (control) or Trimetazidine (TMZ) (**B**) Mass spectrometry lipidomic analysis of fatty-acids accumulating in H460 cells (OX-) cells treated with saline (control) or Trimetazidine (TMZ) during 48 hours.
**FIGURE 15****:** Mass spectrometry lipidomic analysis on the blood of OX+LUAD mouse treated with saline or trimetazidine (TMZ) 20mg/kg/day (at day 7).
**FIGURE 16****:** Respiration of A549 (OX+) cells treated with saline (control) or 500µM Trimetazidine (TMZ) for 48H. Routine, FCCP-uncoupled (ETS) or rotenone insensitive (ROX) respiratory rates determined by high-resolution respirometry.
**FIGURE 17****:** Measurement of the NAD+/NADH ratio in A549 (OX+) cells treated with 500µM Trimetazidine (TMZ) for 48H.
**FIGURE 18****:** Total cellular ATP content of A549 cells treated with saline or 500µMTrimetazidine (TMZ) for 48H.
**FIGURE 19****:** NADH-oxidizing complex I enzymatic activity determined spectrophotometrically on A549 (OX+) cells treated with 500 µM Trimetazidine (TMZ) during 48H or on cell homogenate treated with 100 µM palmitoyl-Coa (10 min incubation).
**FIGURE 20****:** HADHA expression H-score in tumors obtained from LUAD patients with variable FDG-PET-scan SUVmax values.
**FIGURE 21****:** (**A**) List of proteins and phosphorylated proteins significantly reduced in content in the HADHA+ population (TCGA proteome data). It shows a decrease in AKT and GS3KBeta active (phosphorylated) proteins that are negative regulators of HADHA expression. (B) Scheme of Beta catenin signaling pathway showing the control of HADHA expression by Akt, GSK3beta and beta catenin.
**FIGURE 22****:** (A) Drug-sensitivity to beta-catenin inhibitor MSAB on A549 (OX+) or H460 (OX-) cell lines. (B) Positive regulation of HADHA expression by GS3KB inhibitor -betacatenin activator LiCl, 20 mM in A549 (OX+) cells.
**FIGURE 23****:** Cancer-killing effect of ranolazine on A549 cells in vitro.
**FIGURE 24****:** TMZ mode of action on complex I inhibition. (A) Destabilization of immunocaptured complex I composition by shHADHA in A549 cells (loss of CI assembly factor CIA30 - 47KDa on immunocaptured complex I). (B and C) Destabilization of complex I subunits by shHADHA and TMZ (verification by proteomics).
**FIGURE 25****:** Effect of shHADHA and TMZ on lung cancer on an orthotopic mouse model of human LUAD in vivo. (A) *in vivo* tumor size determination using luminescence imaging of A549 cells expressing luciferase at day 3 and day 18 in four groups: shscramble, shHADHA, saline and TMZ. (B) Reduction of tumor size by shHADHA (C) Reduction of tumor size by TMZ 20mg/kg/day

### DETAILED DESCRIPTION

The present invention generally provides MTP inhibitor for use in the treatment of a bioenergetic subgroup of tumors with high respiration (OX+).

Hence, the present invention related to MTP inhibitor for use in the treatment of a high mitochondrial respiration (OX+) cancer.

The terms "high mitochondrial respiration cancer" or "high mitochondrial respiration cancer (OX+)" or "OX+" or "OX+ tumors" relate to tumors or cancers comprising tumors that rely predominantly on mitochondrial respiration to transduce energy.

By contrast, "low mitochondrial respiration cancer" or "low mitochondrial respiration (OX-) cancer" or "OX-" or "OX- tumors" relate to tumors or cancer comprising tumors that do not rely on mitochondrial respiration to transduce energy.

Hence, the treatment of a high mitochondrial respiration (OX+) cancer relates to the treatment of cancer that rely partly on mitochondrial respiration to transduce energy.

The inventors further discover that high mitochondrial respiration OX+ tumors are associated with a low mitochondrial respiration adjacent non-cancer tissue. In return, low mitochondrial respiration OX- tumors are associated with high mitochondrial respiration non-cancer adjacent tissue. The inventors observed that respiration of the adjacent tumor systematically opposed respiration of the tumor region, identifying the concept of "bioenergetic mispairing".

As used herein, the "adjacent tissue" or "cancer adjacent tissue" (CAT) or "adjacent tumor" or "healthy tissue (HT)" is the healthy tissue surrounding the tumor (within margin).

It falls within the ability of the skilled artisan to carry out a method for measuring mitochondrial respiration.

The mitochondrial respiration can be measured by High Resolution Respirometry (HRR). Typically, HRR is measured with a high-resolution oxygraph. The principle of closed-chamber HRR is based on monitoring oxygen concentration in the incubation medium over time, and plotting oxygen consumption by the biological sample (J_{O2}) while performing the various titrations in a respirometric protocol. Protocol is for example described in Pesta and al. "High-Resolution Respirometry: OXPHOS Protocols for Human Cells and Permeabilized Fibers from Small Biopsies of Human Muscle" Methods in Molecular Biology,vol. 810, DOI 10.1007/978-1-61779-382-0_3.

Typically, Oxygraph-2k (O2k, OROBOROS Instruments, Innsbruck, Austria) can be used.

In one embodiment, the mitochondrial respiration is measured on tumors.

In another embodiment, the mitochondrial respiration is measured on the adjacent tissue.

In still another embodiment, the mitochondrial respiration of the tumors is compared to the mitochondrial respiration of the adjacent tissue in order to evidence a group with high respiratory rate (OX+) and a group with low respiratory rate (OX-).

As used herein, and on an indicative basis, values for "high respiratory rate" or "low respiratory rates" can be defined in two manners: i) relative to the respiratory rate of the adjacent tissue or *ii*) in absolute values.

In the relative terms, "high respiratory rates" are higher than 150% of the respiratory rate of the adjacent tissue and values for "low respiratory rate" are lower than 50% of the respiration of the adjacent tissue. In absolute terms, "high respiratory rate" are higher than 0.5 µmol O/min/mg dry weight and values for "low respiratory rate" are lower than 0.5 µmol O/min/mg dry weight.

It has also been determined that high mitochondrial respiration tumor (OX+) poorly incorporated [¹⁸F]fluorodeoxy-glucose compared to the adjacent tissue.

Hence, the bioenergetic subgroup of tumor with high respiration (OX+) is further a subgroup of tumor with low glucose incorporation (FDG-).

The present invention also relates to MTP inhibitor for use in the treatment of high mitochondrial respiration (OX+) cancer, wherein the OX+ cancer is also a low glucose incorporation (FDG-) cancer.

As used herein, and on an indicative basis, values for "low glucose incorporation" are SUVmax (Standardized Uptake) values lower than 10.

The one skilled in the art known which method using in order to measure [¹⁸F]fluorodeoxy-glucose.

Typically, Positron Emission Tomography (PET) can be used. A common use for PET is to measure the rate of consumption of glucose in different parts of the body. Positron emission tomography (PET) is a technique that measures physiological function by looking at blood flow, metabolism, neurotransmitters, and radiolabelled drugs. The technique is based on the detection of radioactivity emitted after a small amount of a radioactive tracer is injected into a peripheral vein. The tracer is administered as an intravenous injection usually labelled with oxygen-15, fluorine-18, carbon-11, or nitrogen-13. The total radioactive dose is similar to the dose used in computed tomography.

Accumulation of the radiolabeled glucose analogue 18-fluorodeoxyglucose (FDG) allows measurement of the rate of consumption of glucose.

It has also been determined that high mitochondrial respiration tumor (OX+) overexpress MTP (subunits alpha (HADHA) and beta (HADHB)) compared to the adjacent tissue.

The bioenergetic subgroup of tumor with high respiration (OX+) is further a subgroup of tumor with high Mitochondrial Trifunctional Protein expression (MTP+), i.e., with high HADHA and/or HADHB expression (HADHA+ and/or HADHB+).

The present invention also relates to MTP inhibitor for use in the treatment of high mitochondrial respiration (OX+) cancer, wherein the OX+ cancer is a high Mitochondrial Trifunctional Protein expression (MTP+).

Alternatively, measurement of MTP expression (subunits HADHA and/or HADHB) in tumors and adjacent tissue can also allow distinguishing a high mitochondrial respiration cancer (OX+) from a low mitochondrial respiration cancer (OX-).

The one skilled in the art knows which method used in order to measure MTP (subunits alpha (HADHA) and/or beta (HADHB)) expression. The level can be determined in lung tissue and/or adjacent tissue. For example, MTP expression can be measured by immunohistology using a HADHA and/or a HADHB antibody, western blot on a tumor biopsy protein lysate or QPCR on a tumor biopsy RNA extract or mass spectrometry proteomics on tumor biopsy protein lysate.

For example, the primers used to measure HADHA expression by QPCR were:
HUMAN HADHA_F - sigma HA08343578 - ACTAAAACCTCCAGA GGAAC (SEQ ID NO: 1) HUMAN HADHA_R - sigma HA08343579 - GTCAATTTTTCCACCAATCC (SEQ ID NO: 2)

The MTP (subunits alpha (HADHA) and/or beta (HADHB)) expression can also be measured by calculating H-score.

HADHA histo score (Hscore) was defined as follow: First, the integrated density of HADHA staining (pixels intensity x pixels area) was measured in a defined window within the cancer region. Then, this result was normalized to the area stained by hematoxylin (cell nuclei) within the same window. This score gives a measure of HADHA staining per nuclei number (Histo-score). For each tumor samples three different windows were analyzed on three different sections.

As used herein and on an indicative basis, values for a "high Mitochondrial trifunctional protein expression" are defined by H-score > 86.

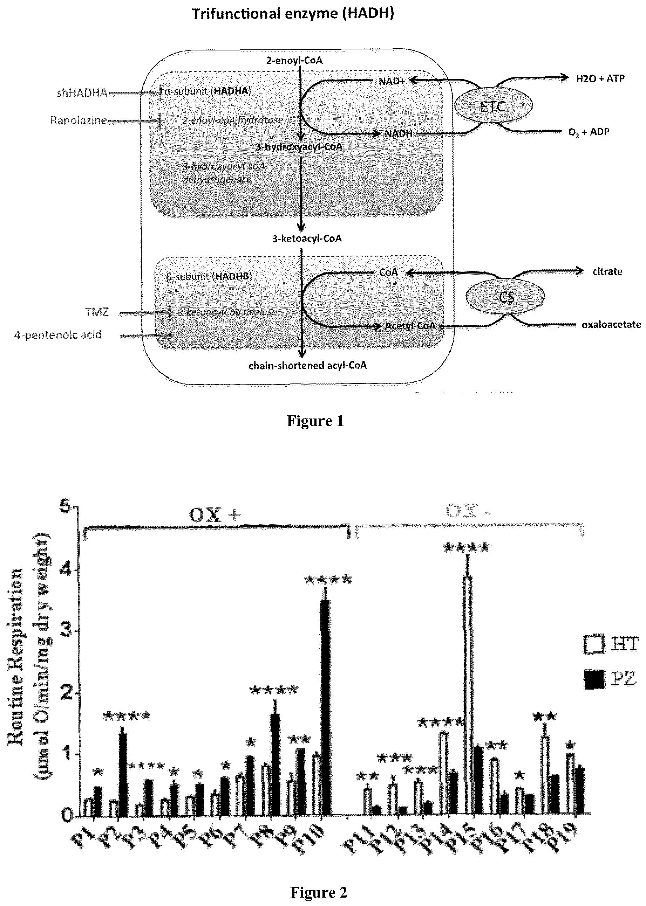

Mitochondrial Trifunctional Protein (MTP) is a heterotrimeric protein that consists of α-subunits (HADHA) and β-subunits (HADHB) and catalyzes three of the four chain-shortening reactions in mitochondrial β-oxidation as described in Figure 1.

The expression of "MTP inhibitor" should be understood broadly, this expression refers to any agents altering with the catalytic activities of the mitochondrial trifunctional protein enzyme complex "MTP" (including alpha and beta subunits denominated HADHA and HADHB) or down-regulating the expression of MTP alpha and/or beta subunit or destabilizing the enzyme complex.

In one embodiment, the MTP inhibitor is a HADHA inhibitor for use in the treatment of a high mitochondrial respiration (OX+) cancer.

In another embodiment, the MTP inhibitor is a HADHB inhibitor for use in the treatment of a high mitochondrial respiration (OX+) cancer.

In one embodiment of the present invention, the inhibitor of MTP is an agent down-regulating the expression of MTP in OX+ tumors. Typically, agent down-regulating the expression of MTP comprises a nucleic acid which interferes with the expression of MTP or a pharmacological modulator of MTP transcriptional regulators.

Examples of such agents are antisense molecules or vectors comprising said antisense molecules. Antisense molecules are complementary strands of small segments of mRNA. Methods for designing effective antisense molecules being well known (see for example US6165990), and it falls within the ability of the skilled artisan to design antisense molecules able to downregulate the expression of MTP in OX+ tumors. Further examples are RNA interference (RNAi) molecules such as, for example, short interfering RNAs (siRNAs) and short hairpin RNAs (shRNAs). RNAi refers to the introduction of homologous double stranded RNA to specifically target a gene's product, in the present case MTP, resulting in a null or hypomorphic phenotype. Methods for designing effective RNAi molecules being well known (see for review Hannon and Rossi Nature. 2004 Sep 16; 431(7006):371-8), it falls within the ability of the skilled artisan to design RNAi molecules able to downregulate the expression of MTP in OX+ tumors.

Examples of siRNAs able to downregulate the expression of HADHA in OX+ tumors are nucleic acid molecules which comprise one of the following sequences:
The shRNA targeing HADHA was obtained from OriGene catalog # TL312528 - HADHA - human sh RNA constructs in lentiviral GFP vector - A549 shHADHA - AAGCCTATTGTGGCTGCCATCAATGGATC (SEQ ID NO: 3).

The control shRNA was: OriGene catalog # TR30021 - A549 shScramble - HuSH 29-mer Non-Effective shRNA Scrambled Cassette

In one preferred embodiment, the agent down-regulating the expression of HADHA in OX+ tumors is shHADHA which alters with the catalytic activity of α-subunit (HADHA) of the mitochondrial trifunctional protein.

In another embodiment of the invention, MTP inhibitor can be Trimetazidine (1-(2,3,4-trimethoxybenzyl)piperazine) or Ranolazine ((±)-N-(2,6-dimethylphenyl)-4-[2-hydroxy-3-(2-methoxyphenoxy)-propyl]-1-piperazineacetamide) or 4-pentenoic acid.

Ranolazine inhibits the mitochondrial trifunctional protein and more particularly Ranolazine reduces the catalytic activity of α-subunit (HADHA) of the mitochondrial trifunctional protein.

Trimetazidine and 4-pentenoic acid inhibit the mitochondrial trifunctional protein and more particularly Trimetazidine and 4-pentenoic acid reduce the catalytic activity of β-subunit (HADHB).

Preferably, the MTP inhibitor is a HADHA inhibitor.

Preferably, the MTP inhibitor is Trimetazidine.

The above defined MTP inhibitor, and in particular Trimetazidine may be used for treating any high mitochondrial respiration cancer (OX+) because of the mode of action of MTP inhibitors, and in particular Trimetazidine impact the activity of respiratory chain complex I. Indeed, most high mitochondrial respiration cancers (OX+) rely predominantly on the mitochondrial respiratory chain function to transduce energy.

For example, high mitochondrial respiration (OX+) cancer and low mitochondrial respiration (OX-) cancer sub-groups have been identified in prostate cancer (*Oxidative phosphorylation and mitochondrial function differ between human prostate tissue and cultured cells.* Schöpf B, Schäfer G, Weber A, Talasz H, Eder IE, Klocker H, Gnaiger E. FEBS J. 2016 Jun;283(11):2181-96.), in B-lymphomas (Metabolic signatures uncover distinct targets in molecular subsets of diffuse large B cell lymphoma. Caro P, Kishan AU, Norberg E, Stanley IA, Chapuy B, Ficarro SB, Polak K, Tondera D, Gounarides J, Yin H, Zhou F, Green MR, Chen L, Monti S, Marto JA, Shipp MA, Danial NN. Cancer Cell. 2012 Oct 16;22(4):547-60), in leukemia's (Targeting mitochondrial oxidative phosphorylation eradicates therapy-resistant chronic myeloid leukemia stem cells. Kuntz EM, Baquero P, Michie AM, Dunn K, Tardito S, Holyoake TL, Helgason GV, Gottlieb E. Nat Med. 2017 Sep 18. doi: 10.1038/nm.4399; Pharmacologic inhibition of fatty acid oxidation sensitizes human leukemia cells to apoptosis induction, J Clin Invest. 2010 Jan;120(1):142-56. doi: 10.1172/JCI38942. Epub 2009 Dec 21; Chemotherapy-Resistant Human Acute Myeloid Leukemia Cells Are Not Enriched for Leukemic Stem Cells but Require Oxidative Metabolism, Samudio I1, Harmancey R, Fiegl M, Kantarjian H, Konopleva M, Korchin B, Kaluarachchi K, Bornmann W, Duvvuri S, Taegtmeyer H, Andreeff M.; Cancer Discov. 2017 Jul;7(7):716-735. doi: 10.1158/2159-8290.CD-16-0441. Epub 2017 Apr 17) and in melanomas (PGC1α expression defines a subset of human melanoma tumors with increased mitochondrial capacity and resistance to oxidative stress, Vazquez F1, Lim JH, Chim H, Bhalla K, Girnun G, Pierce K, Clish CB, Granter SR, Widlund HR, Spiegelman BM, Puigserver P, Cancer Cell. 2013 Mar 18;23(3):287-301. doi: 10.1016/j.ccr.2012.11.020. Epub 2013 Feb 14), in ovarian tumors (*""*Metabolic reprogramming" in ovarian cancer cells resistant to cisplatin", Montopoli M, Bellanda M, Lonardoni F, Ragazzi E, Dorigo P, Froldi G, Mammi S, Caparrotta L. Curr Cancer Drug Targets. 2011 Feb;11(2):226-35.), in breast tumors (*"*Inhibition of fatty acid oxidation as a therapy for MYC-overexpressing triple-negative breast cancer", Camarda R, Zhou AY, Kohnz RA, Balakrishnan S, Mahieu C, Anderton B, Eyob H, Kajimura S, Tward A, Krings G, Nomura DK, Goga A. Nat Med. 2016 Apr;22(4):427-32.), in sarcomas (*"*Bioenergetic properties of human sarcoma cells help define sensitivity to metabolic inhibitors" Sameer H Issaq, Beverly A Teicher, and Anne Monks. Cell Cycle. 2014 Apr 1; 13(7): 1152-1161.) in kidney cancers (*"*Suppression of mitochondrial respiration with auraptene inhibits the progression of renal cell carcinoma: involvement of HIF-1α degradation", Jang Y, Han J, Kim SJ, Kim J, Lee MJ, Jeong S, Ryu MJ, Seo KS, Choi SY, Shong M, Lim K, Heo JY, Kweon GR. Oncotarget. 2015 Nov 10;6(35):38127-38), and in hepatomas (*"*Energy metabolism determines the sensitivity of human hepatocellular carcinoma cells to mitochondrial inhibitors and biguanide drugs". Hsu CC, Wu LC, Hsia CY, Yin PH, Chi CW, Yeh TS, Lee HC. Oncol Rep. 2015 Sep;34(3): 1620-8.)

Hence, in a preferred embodiment, the high mitochondrial respiration cancer (OX+) is chosen among prostate cancer, lymphomas, leukemia, melanomas, ovarian tumors, breast tumors, kidney cancers, hepatomas and lung cancers.

Preferably, high mitochondrial respiration (OX+) cancer is lung cancer, preferably non-small cell lung cancer.

In a particularly preferred embodiment, the cancer is a high mitochondrial respiration (OX+) lung adenocarcinoma.

Hence, and as used herein, a "high mitochondrial respiration (OX+) lung adenocarcinoma" is a lung adenocarcinoma that relies predominantly on mitochondrial function to transduce energy. As a consequence, mitochondrial inhibition alters tumor growth and survival.

In the context of the invention, the term "treating" or "treatment" or "method of treating" or its equivalent is not intended as an absolute term and, as used herein, refer to a procedure or course of action that is design for reversing, alleviating, inhibiting the progress of, or preventing the disorder or condition to which such term applies, or reversing, alleviating, inhibiting the progress of, or preventing one or more symptoms of high mitochondrial respiration cancer. A "treatment" or a "method of treating" high mitochondrial respiration cancer does not necessarily mean that the high mitochondrial respiration cancer will, in fact, be eliminated, be reduced, or that the symptoms of high mitochondrial respiration cancer will, in fact be alleviated. Often, a "treatment" or a "method of treating" high mitochondrial respiration cancer will be performed even with a low likelihood of success but is nevertheless deemed to induce an overall beneficial course of action.

The inventors discovered that some OX+ tumors overexpress fatty-acid oxidation (FAO) proteins and use fatty-acid as energy source for their growth, survival and resistance to treatments or activation of metastatic processes. These cancers are hence, and as used herein "high fatty acid oxidation (FAO+) cancers" or "high fatty acid oxidation cancers", or "FAO+" or "FAO+" tumors.

In one embodiment, the high mitochondrial respiration (OX+) cancer is further a high fatty-acid oxidation (FAO+) cancer.

Typically, the high mitochondrial respiration (OX+) and high fatty-acid oxidation (FAO+) cancer is chosen among lung cancer or lymphoma.

It has also been described that lymphoma overexpress fatty-acid oxidation (FAO) and use fatty-acid as energy source for their growth, survival and resistance to treatments or activation of metastatic processes (*"*Metabolic Signatures Uncover Distinct Targets in Molecular Subsets of Diffuse Large B-Cell Lymphoma" Pilar Caro, Amar U. Kishan,Erik Norberg, Illana Stanley, Bjoern Chapuy, Scott B. Ficarro, Klaudia Polak, Daniel Tondera, John Gounarides, Hong Yin, Feng Zhou,Michael R. Green, Linfeng Chen, Stefano Monti, Jarrod A. Marto, Margaret A. Shipp, and Nika N. Danial Cancer Cell. 2012 Oct 16; 22(4): 547-560).

Preferably, the high mitochondrial respiration (OX+) and high fatty-acid oxidation (FAO+) cancer is lung adenocarcinoma (LUAD).

It falls within the ability of the skilled artisan to carry out a method for measuring FAO+.

Typically, high fatty-acid oxidation (FAO+) can be measured by following the incorporation of palmitate in Krebs cycle at the level of citrate using mass spectrometry.

In another embodiment, the high mitochondrial respiration (OX+) cancer is further a nPA + cancer. As used herein, "nPA" means "number of cigarette pack-years. The inventors discover that accumulated exposure to tobacco smoke quantified by the number of cigarette pack-years (nPA) showed a significantly higher value in the OX+ patients (p=0.0129).

The MTP inhibitor according to the invention may be used in combination any other therapeutic strategy for treating cancer e.g. surgery, external radiotherapy, chemotherapy.

It has also been discovered that MTP overexpression depends on beta-catenin activation since OX+ tumors were enriched in beta-catenin and since beta-catenin modulation altered HADHA expression *in vitro.*

In a preferred embodiment, the MTP inhibitor is used in combination with a beta-catenin inhibitor.

Typically, the beta-catenin inhibitor can be small-molecule inhibitors capable of disrupting TCF/β-catenin complexes such as PKF118-310, CGP049090, PFK115-584, PFK222-815, PKF118-744, or ICG001.
It can belong to the class of β-catenin-responsive transcription inhibitors such as iCRT, 3, 5, 14, or NCO4314.

The beta-catenin inhibitor can also target b-catenin-CBP interactions such as ICG-001 an inhibitor of beta-catenin/CREB-binding protein transcription activity, or target

The beta catenin inhibitor can be chosen among CCT036477 capable of inhibiting TCF/b-catenin-mediated transcription and inducing cancer cell death, or XAV939 which antagonized Wnt/b-catenin pathway by inhibiting tankyrase, or a special class of compounds, acyl hydrazones with iron chelating activity which have an inhibitory effect on the Wnt/b-catenin signaling pathway linked to iron chelation or N-((8-hydroxy-7-quinolinyl) (4-methylphenyl)methyl)benzamide (HQBA).

It can also be selected among molecules with 2,3,6-trisubstitued pyrido[2,3-b] pyrazine core skeletons, or carnosic acid or CCT031374 or Ibuprofen or aspirin.

These beta-catenin inhibitors are described in Thakur and Al, "Pharmacological modulation of beta-catenin and its applications in cancer therapy", J.Cell.Mol.Med. Vol 17, No 4, 2013 pp. 449-456, January 17, 2013.

Preferably, the beta-catenin inhibitor is MSAB (methyl 3-{[(4-methylphenyl)sulfonyl]amino}benzoate) which is a selective inhibitor of Wnt/(β-catenin signaling.

In another embodiment, the beta-catenin inhibitor can be an alpha-helix mimetic structure.

In another embodiment of the invention the HADHA inhibitor is used in combination with metformin.

In another embodiment of the present invention, and before using a MTP inhibitor for the treatment of high mitochondrial respiration (OX+) cancers, a diagnostic test may be performed in order to determine the expression of MTP (subunits alpha (HADHA) and/or beta (HADHB)) in tumors. By performing such a pre-treatment diagnostic test, it is possible to determine whether a subject would be responsive to a method of treatment according to the invention.

By performing such pre-treatment diagnostic test, it is also possible to determine the prognosis of a subject suffering from cancers, comprising the step of measuring the expression of MTP in the tumor.

The inventors surprisingly discovered that MTP (subunits alpha (HADHA) and beta (HADHB)) expression in tumors varied in negative correlation with [¹⁸F]fluorodeoxy-glucose incorporation.

More particularly, HADHA expression in tumors varied in negative correlation with [¹⁸F]-FDG fluorodeoxy-glucose incorporation.

Hence, a further step of measuring [¹⁸F]fluorodeoxy-glucose incorporation can be added.

A further step of determining the number of cigarette pack-years (nPA) can be added as a marker to determine the prognosis of a subject suffering from cancers.

Hence, the present invention also relates to an *in vitro* method of determining the prognosis of a subject suffering from high mitochondria respiration cancers comprising the step of measuring the expression of MTP in tumors. The method can include of further step of measuring [¹⁸F] fluorodeoxy-glucose incorporation.

A high expression of MTP in tumors is indicative of poor prognosis.

Alternatively, levels of MTP in a sample obtained from the subjects may be determined in order to treat only those subjects having an elevated level of MTP in tumor samples.

It falls within the ability of the skilled artisan to carry out such a diagnostic test.

The present invention also covers a pharmaceutical composition comprising at least a MTP inhibitor.

Pharmaceutical composition encompassed by the present invention can further include one or more pharmaceutically acceptable carriers.

A used herein, "pharmaceutically acceptable" refers to those compounds, materials, excipients, compositions or dosage forms which are, within the scope of sound medical judgment suitable for use in contact with the tissues of subjects, without excessive toxicity, irritation, allergic response or other problem complications commensurate with a reasonable benefit/risk ratio. Pharmaceutically acceptable carriers include but are not limited to solvents, dispersion media, coatings, stabilizing agents, diluents, preservatives, antibacterial and antifungal agents, isotonic agents, adsorption delaying agents, and the like. Diluents can include water, saline, dextrose, ethanol, glycerol and the like. Isotonic agents can include sodium chloride, dextrose, mannitol, sorbitol and lactose, among others known to those skilled in the art. Stabilizers include albumin. Preservatives include merthiolate.

In a preferred embodiment, the pharmaceutical composition is for use for treating high mitochondrial respiration (OX+) cancers as described above.

In another embodiment, the pharmaceutical composition is for use for treating high mitochondrial respiration (OX+) and high fatty-oxidation (FAO+) cancers as described above.

The present invention also concerns a method for the treatment of high mitochondrial respiration (OX+) cancers as described above in a subject in need thereof comprising administering to said subject an effective amount of a MTP inhibitor.

The present invention also concerns a method for the treatment of a high mitochondrial respiration (OX+) and high fatty-acid oxidation (FAO+) cancers as described above in a subject in need thereof comprising administering to said subject an effective amount of a MTP inhibitor.

All the embodiment disclosed above are encompassed in these aspects.

By an "effective amount" of an inhibitor of the MTP is meant a sufficient amount to treat high respiration mitochondrial (OX+) cancers, preferably high respiration mitochondrial (OX+) and high fatty-acid oxidation (FAO+) cancers, at a reasonable benefit/risk ratio applicable to any medical treatment. It will be understood, however, that the total daily usage of the inhibitor of MTP will be decided by the attending physician within the scope of sound medical judgment. The specific therapeutically effective dose level for any particular subject in need thereof will depend upon a variety of factors including the stage of high respiration mitochondrial (OX+) cancer being treated and the activity of the specific MTP inhibitor employed, the age, body weight, general health, sex and diet of the subject, the time of administration, route of administration, the duration of the treatment; drugs used in combination or coincidental with the and like factors well known in the medical arts. For example, it is well known within the skill of the art to start doses of the compound at levels lower than those required to achieve the desired therapeutic effect and to gradually increase the dosage until the desired effect is achieved.

Typically medicaments according to the invention comprise a MTP inhibitor, together with a pharmaceutically-acceptable carrier. A person skilled in the art will be aware of suitable carriers. Suitable formulations for administration by any desired route may be prepared by standard methods, for example by reference to well-known text such as Remington; The Science and Practice of Pharmacy.

In another aspect, the present inventions relates to the use of HADHA inhibitor for the manufacture of a pharmaceutical preparation for the treatment of high mitochondrial respiration (OX+) cancers, and more preferably for the treatment of high mitochondrial respiration (OX+) and high fatty-acid oxidation (FAO+) cancers.

All the embodiment disclosed above are encompassed in these aspects.

### EXAMPLES

In the following description, all molecular biology experiments for which no detailed protocol is given are performed according to standard protocol.

### Summary

In the following examples, a functional analysis of mitochondrial activity on freshly excised human lung adenocarcinoma surgical pieces was performed and allowed to identify two bioenergetics subsets of tumors: OX+ and OX- (Example 1).

Comparative study using label-free proteomics was performed in order to investigate the molecular basis of the bioenergetics differences between the OX+ and the OX- tumors (Example 2).

These findings were used to validate a molecular signature for OX+ lung tumors and to identify a potential metabolic vulnerability (Example 3).

The validity of inhibiting the mitochondrial trifunctional protein enzyme complex (MTP) in OX+ lung tumors was demonstrated and a preclinical study using an approved MTP inhibitor, trimetazidine, was performed (example 4).

The mode of action of the inhibitor revealed a combined inhibition of oxidative phosphorylation at the level of MTP and respiratory chain complex I, leading to a cellular energy crisis and redox imbalance (example 5).

MTP expression was high in OX+ tumors in negative correlation with their capacity to accumulate glucose, as assessed by the FDG-PET scan SUVmax value (example 6).

Finally, it has been demonstrated that Ranolazine, another MTP inhibitor, reduces A549 cell viability (example 7).

Altogether, these findings provide proof-of-concept data for bioenergetic-based precision medicine and for the use of MTP inhibitors for the treatment of high mitochondrial respiration cancers (OX+).

The following materials and methods were used in the examples:

### Human lung tumors sampling

Surgical pieces of lung adenocarcinoma were obtained from the Hospital of Haut-Lévèque, CHU of Bordeaux. The surgeon performed tumor resection and the pathologist collected thereafter the two areas of interest for the study: healthy tumor-adjacent tissue (HT) and cancer proliferating zone (PZ; Ox+ or Ox-). Utilization of these tissues for research purpose was approved by the ethics committee (Comité de Protection des Personnes Sud-Ouest et Outre Mer III). Immediately after tumor excision and sample preparation by the pathologist the tissues were analyzed for mitochondrial respiration or frozen for molecular analyses as detailed below.

### High-Resolution Respirometry (HRR)

Routine respiration that was sensitive to respiratory chain inhibition by cyanide was measured in tissues using high-resolution respirometry (HRR) in DMEM supplemented with 10% FCS. High-resolution respirometry is used for measuring mitochondrial respiration on intact lung tumors (PZ) and paired-adjacent tissue (CAT or HT). This method was originally developed for the evaluation of mitochondrial respiration in muscle biopsies for the diagnosis of mitochondrial diseases and was herein adapted to the study of human lung tumors. In particular, it has been observed that no permeabilization of the tissue was required to assess mitochondrial respiration and to obtain inhibition by oligomycin and KCN. Each piece of lung tumor or paired-adjacent tissue was divided in 3 to 5 samples of 10-20 mg and respiration was evaluated at 37°C in the O₂k (Oroboros) apparatus.

The respiratory medium consisted of low-glucose (5mM) DMEM supplemented with 10% FCS. To assess the endogenous respiratory coupling ratio (RCR) 20µg/µl oligomycin (Sigma-Aldrich) were used and to exclude non-mitochondrial respiration 2mM KCN were used. The rate of respiration was normalized to the dry-weight of the lung tumor biopsy.

### Immunohistochemistry and image analysis (Mice and human)

Surgical specimens were fixed in 10% buffered formaldehyde and tissue sections from tumoral and non tumoral parenchyma were routinely processed for the purpose of diagnosis. Selected representative slides including tumoral and non tumoral tissue were processed for HADHA immunohistochemistry (IHC). The 2.5µm thick sections were dewaxed and rehydrated and antigen retrieval was performed in a 1mM Tris- EDTA pH9 solution. All staining procedures were performed in an automated autostainer (Dako-Agilent Clara, United States) using standard reagents provided by the manufacturer. The sections were incubated with the HADHA (Abcam EPR17940) at a 1:1000 dilution for 45 min at room temperature. EnVision Flex/HRP (Horseradish peroxidase) (Dako-Agilent, 20 minutes) was used for signal amplification. 3,3'-Diamino-benzidine (DAB, Dako) development was used for detecting primary antibodies. The slides were counterstained with hematoxylin, dehydrated and mounted. Each immunohistochemical run contained a negative control (buffer, no primary antibody). Sections were visualized with a Nikon-Eclipse501 microscope, and images were acquired using NIS-Elements F.

### Positron Emission Tomography (PET) to measure the rate of consumption of glucose

For FDG-PET/CT, a 4D acquisition was performed in radiotherapy treatment position, 50 min after intravenously injection of 3.7 MBq/Kg of [18F]-FDG, on a PET/CT integrated system (Discovery RX, General Electric Medical System®, Milwaukee, WI) using the Real-time Position Management (RPM) device (Varian®) and the "Motion Free" software (General Electric Medical System®). The respiratory cycle was rebinned in six phases.

### Human tumors Proteomics

### Sample preparation and protein digestion

10 µg of each protein sample were solubilized in Laemlli buffer and were deposited onto SDS-PAGE gel. After colloidal blue staining, bands were cut in 8 equal bands, each band being subsequently cut again in 1 mm x 1 mm gel pieces. Gel pieces were detained in 25 mM ammonium bicarbonate 50% ACN, rinsed twice in ultrapure water and shrunk in ACN for 10 min. After ACN removal, gel pieces were dried at room temperature, covered with the trypsin solution (10 ng/µl in 40 mM NH₄HCO₃ and 10% ACN), rehydrated at 4 °C for 10 min, and finally incubated overnight at 37 °C. Spots were then incubated for 15 min in 40 mM NH₄HCO₃ and 10% ACN at room temperature with rotary shaking. The supernatant was collected, and an H₂O/ACN/HCOOH (47.5:47.5:5) extraction solution was added onto gel slices for 15 min. The extraction step was repeated twice. Supernatants were dried in a vacuum centrifuge and suspended in 100 µL H₂O/HCOOH (100:0.1).

### nLC-MS/MS analysis

Peptide mixture was analyzed on a Ultimate 3000 nanoLC system (Dionex) coupled to a nanospray LTQ-Orbitrap XL mass spectrometer (ThermoFinnigan, San Jose, CA). Ten microliters of peptide digests were loaded onto a 300-µm-inner diameter x 5-mm C₁₈ PepMap™ trap column (LC Packings) at a flow rate of 20 µL/min. The peptides were eluted from the trap column onto an analytical 75-mm id x 15-cm C18 Pep-Map column (LC Packings) with a 5-40% linear gradient of solvent B in 105 min (solvent A was 0.1% formic acid in 5% ACN, and solvent B was 0.1% formic acid in 80% ACN). The separation flow rate was set at 200 nL/min. The mass spectrometer operated in positive ion mode at a 1.8-kV needle voltage and a 47-V capillary voltage. Data were acquired in a data-dependent mode. MS scans (m/z 300-1700) were recorded at a resolution of R = 60 000 (@ m/z 400) and an AGC target of 5 x 10⁵ ions collected within 500 ms. Dynamic exclusion was up to 30 s and top 6 ions were selected from fragmentation in CID mode. MS/MS scans with a target value of 1 x 10⁴ ions were collected with a maximum fill time of 200 ms.

### Database search and results processing

Data were searched by SEQUEST through Proteome Discoverer 1.4 (Thermo Fisher Scientific Inc.) against a subset of the 2012.09 version of UniProt database restricted to Human Reference Proteome Set (67,949 entries). Spectra from peptides higher than 5000 Da or lower than 350 Da were rejected. The search parameters were as follows: mass accuracy of the monoisotopic peptide precursor and peptide fragments was set to 10 ppm and 0.6 Da respectively. Only b- and y-ions were considered for mass calculation. Oxidation of methionines (+16 Da) and carbamidomethylation of cysteines (+57 Da) were considered as variable modifications. Two missed trypsin cleavages were allowed. Peptide validation was performed using Percolator algorithm and only "high confidence" peptides were retained corresponding to a 1% False Positive Rate at peptide level.

### Label-Free Quantitative Data Analysis

Raw LC-MS/MS data were imported in Progenesis LC-MS 4.0 (Nonlinear Dynamics Ltd, Newcastle, U.K). Data processing includes the following steps: (i) Features detection, (ii) Features alignment across the 12 sample, (iii) Volume integration for 2-6 charge-state ions, (iv) Normalization on total protein abundance, (v) Import of sequence information, (vi) ANOVA test at peptide level and filtering for features p <0.05, (vii) Calculation of protein abundance (sum of the volume of corresponding peptides), (viii) ANOVA test at protein level and filtering for features p <0.05. Noticeably, only non-conflicting features and unique peptides were considered for calculation at protein level. Quantitative data were considered for proteins quantified by a minimum of 2 peptides. The volcano plots analysis were performed using GraphPad Prism 6 (GraphPad Software, inc.).

### Bioinformatic analysis of human LUAD samples from TCGA.

Level 3 released gene level expression data for RNAseq were retrieved for 430 lung adenocarcinomas from The Cancer Genome Atlas (TCGA ; https://tcga-data.nci.nih.gov). The data processing, quality control and normalization were done by TCGA workgroup. Normalized RSEM (RNAseq by Expectation-Maximization) values were used for the analysis. A hierarchical clustering was applied using the euclidean distance and the Ward method for aggregation using the R package EMA.

Kaplan-Meier curves were performed using the R package Survival. Log-rank test was performed to test the significant difference between two curves.

### Cell culture conditions

A549_LUC and H460_LUC, human lung cancer cells, were stably transfected with luciferase (pRRLsin-MND-Luc-WPRE). The cells were maintained in DMEM medium (Gibco) 11885-084 and 11965-092 respectively, supplemented with 10% fetal bovine serum (FBS), pH 7.4 at 37°C in a humidified incubation chamber with 5% CO₂. Cells were sub-cultured every 2 days and used on experiments when they reached 85% confluency. Also, they were tested as luciferase positive using direct application of 2 mL of a 150 mg/ml solution of luciferine (Firefly Luciferin, Caliper Lifescience Corp, USA), followed by immediate bioluminescent imaging using the photonIMAGER™ optima system.

### A549-LUC sh HADHA cells

Specific suppression of human HADHA mRNA was achieved by transduction of lentiviral particles expressing oligonucleotides bearing a short hairpin structure (OriGene - TL312528V). A549 cells grown in a 24-well plate at confluence of 50% were incubated with control or HADHA shRNA lentiviral particles. Cells were grown in culture media for 48 h. After washing with PBS, cells were cultured in fresh media for an additional period of 24 h. Cells were then diluted in a 1:5 suspension, and cultured for 10 days in the presence of 1 µg/ml of puromycin dihydrochloride (Sigma- Aldrich) to select resistant cells (shHADHA). Transfected cells with scramble vector were used as negative control and named shScramble. The efficacy of shHADHA was determined by Real Time -qPCR (pré-designers primers Sigma- Aldrich and western blot assay with a specific antibody anti-HADHA (abcam).

### Sulphorodamine B viability assay

Briefly, cells were seeded in 96-well plates and medium was changed after 24 h, then Test Reagent was added on top of the cell wells and incubated for up to 72h. The viability assay was performed using Sulforhodamine B colorimetric assay for viability assay following the protocol described by Vanicha Vichai & Kanyawim Kirtikara.

### Proliferation assay

Briefly, cells were seeded in 96-well plates and medium was changed after 24 h, then Test Reagent (trimetazidine 500 uM) was added on top of the cell wells and incubated for up to 48h. The proliferation assay was performed using the BrdU Cell Proliferation ELISA Kit (colorimetric) according manufacturer's instructions.

### Multicellular Tumor Spheroids (MCTS)

3D cell cultures were prepared essentially as described by Ho et al. Briefly, spheroids were produced by seeding U- bottom wells previously coated with 1% agarose with 2x104 cell suspended in 200 µL DMEM/F-12 medium containing 10% of FBS. With this setup cells do not adhere to the plastic matrix and are thus allowed to associate forming a multicellular 3D structure. The size of the aggregates was monitored every 24 h in the inverted microscope during a total of 72 h.

### ATP measurements

The ATP content was performed using the The CellTiter-Glo® Luminescent Cell Viability Assay (Promega) folowing manufacturer's instructions. Luminescence was read on a spectramax M4 Multi-Mode Microplate Reader (Molecular Devices) and values were calculated based on an ATP standard curve. The assay was normalized to cell number.

### Enzymatic activity assays

Enzymatic activities were measured spectrophotometrically, in homogenate of cells using the following reaction media: Complex I - 50mM potassium phosphate buffer, 100µM Decylubiquinone. The reaction was started by the addition of 100µM NADH and carried out at 37°C for 15 min in 340nm. After the stabilization of the signal was added 12,5µM Rotenone. Complex II - 25mM potassium phosphate buffer, 1mM KCN, 100µM ATP, 50µM DCPIP, 20mM succinate. The reaction was started by the addition of 100µM decylubiquinone and carried out at 37°C for 15 min in 660nm. Complex III - 100mM potassium phosphate buffer, 50µM cytochrome c, 250µM EDTA. The reaction was started by the addition of 100µM decylubiquinol and carried out at 37°C for 15 min in 550nm. After the stabilization of the signal was added 12,5µg/ml antimycin A. Complex IV - 100uM reduced cytochrome C in 50mM potassium phosphate buffer. The reaction was started by the addition of homogenate of cells and carried out at 37°C for 15 min in 550nm.

### NAD/NADH determination

The NAD/NADH was measure using the NAD/NADH Colorimetric assay Kit (abcam), according to the manufacturer's instructions. At 25°C absorbance was measured at 450 nm and the value used to calculate the ratio. The assay was normalized to cell number.

### Western blotting

Cells pellet were lysed in a buffer containing RIPA IX (Sigma-Aldrich) and protease inhibitors cocktail (chymostatin, leupeptin, antipain, pepstatin A - Sigma-Aldrich). Protein lysates were placed on ice for 15 min, vortexed and cleared by centrifugation at 12,000 × g for 15 min at 4 °C. The supernatants were retrieved and frozen at -80 °C until use. Protein concentration was performed using the Pierce™ BCA Protein Assay Kit.

Samples (20ug) were dissolved in Laemmli buffer (Bio-Rad) containing 2% mercaptoethanol by incubation for 15 min at 65°C and separated on a 4-15% SDS-polyacrylamide gradient TGX STAIN FREE mini-gel (Biorad). The gels are activated by UV with chemidoc (Biorad). Proteins were transferred electrophoretically to 0.45 µm of nitrocellulose membranes for 7 min at 2.5 Amp in Transfert buffer with Trans-Blot Turbo (biorad). Membranes were blocked 1h in odyssey buffer (eurobio) and incubated overnight in a mixture of antibodies diluted in odyssey buffer. After three washes with PBS-0.05% Tween 20, the membranes were incubated for 1 h with second antibody (LYCOR) diluted in odyssey buffer. This secondary antibody was detected by fluorescence with odyssey. Proteins were detected using specific antibodies (abcam) and total protein was used as the loading control. Primary antibodies (abcam®) against HADHA, VLCAD, mt-ETFA, mt-ETFB, NDUFS2 and NDUFAB1.

### Oxygen consumption of intact and digitonin-permeabilized lung cancer cells

O₂ consumption rates were measured polarographically using high-resolution respirometry (Oroboros Oxygraph-O2K). After the treatment period, medium was removed and cells were either suspended in DMEM in the presence of glucose or not for measurements of O₂ consumption of intact cells, or in the respiration medium pH 7.2 (mannitol 0.25 M, MgCl₂ 10 mM, KH₂PO₄ 10 mM, HEPES 10 mM, EDTA 0.08 mM, EGTA 1 mM and fatty acid free BSA 0.1%) for evaluation of respiratory complexes of permeabilized cells. Routine consumption, oligomycin-independent respiration (proton leak), FCCP-stimulated respiration (ETS capacity - maximum respiration) and ROX (oxygen consumption in presence of rotenone and antimicyn A) were measured in intact melanoma cells. All the values were corrected by ROX. The respiratory activity of ETF of digitonin 0.0015%-permeabilized lung cancer cells were performed following the addition of palmitoyl-carnitine 25 µM (ETF-linked substrate), rotenone 0.5 µM, ADP 100 µM. DatLab software (Oroboros Instruments, Innsbruck, Austria) was used for data acquisition and analysis.

### RNA extraction and cDNA synthesis

Total RNA was isolated from melanoma cells using QuiaZOL reagent (Quiagen) according to the manufacturer's instructions. Total RNA was quantified spectrophotometrically and 1 µg were treated with 1 U of DNAse RNAse-free (Bio-Rad) cDNA synthesis was performed using the DNAse treated RNA according to iScript cDNA Synthesis kit (Bio-Rad).

### Real Time PCR

Gene expression analysis was performed using CFX96™ Real Time PCR (Bio-Rad) and power SYBR-GREEN PCR master MIX (Applied Biosystems). For this test primer pairs were purchased by pre-desigers primers from Sigma-Aldrich. The comparative Ct method was used to compare changes in gene expression levels. GUSB and RPLP0 were used as an endogenous control.

### Lipidomics

Extracted samples were dissolved in 100 µL CHC13; 15 µL was injected for analysis. LC separation was achieved using a Bio-Bond 5U C4 column (Dikma). The LC solvents were as follows: buffer A, 95:5 H2O:methanol + 0.03% NH4OH; buffer B, 60:35:5 isopropanol:methanol:H2O + 0.03% NH4OH. A typical LC run consisted of the following for 70 min after injection: 0.1 mL/min 100% buffer A for 5 min, 0.4 mL/min linear gradient from 20% buffer B to 100% buffer B over 50 min, 0.5 mL/min 100% buffer B for 8 min and equilibration with 0.5 mL/min 100% buffer A for 7 min. FFA analysis was performed using a Thermo Scientific Q Exactive Plus fitted with a heated electrospray ionization source. The MS source parameters were 4 kV spray voltage, with a probe temperature of 437.5 °C and capillary temperature of 268.75 °C. Full-scan MS data was collected with a resolution of 70 k, AGC target 1 × 106, maximum (max) injection time of 100 ms and a scan range of 150-2,000 m/z. Data-dependent MS (top 5 mode) was acquired with a resolution of 35 k, AGC target 1 × 105, max injection time of 50 ms, isolation window 1 m/z, scan range 200 to 2,000 m/z, stepped normalized collision energy (NCE) of 20, 30 and 40. Extracted ion chromatograms for each FFA were generated using a m/z ± 0.01 mass window around the calculated exact mass (i.e., palmitic acid, calculated exact mass for M-H is 255.2330, and the extracted ion chromatogram was 255.22-255.24). Quantification of the FFAs was performed by measuring the area under the peak and dividing this number by the area under the peak for [¹³C16]palmitate. The ratio of FFA/[13C16]palmitate was then multiplied by 200 pmol (the concentration of the [¹³C16]palmitate) and divided by the tumor weight or plasma volume to afford the molar concentration per unit mass or unit volume.

### Mass spectrometry analysis : Measurement of high fattv-acid oxidation (FAO+)

The incorporation of palmitate in Krebs cycle was followed at the level of citrate using mass spectrometry. A549 cells grown in low glucose DMEM were washed twice with PBS and resuspended in PBS containing 2 mm [U-13C]palmitate with (10 mm) glucose.

The cells were incubated for 2 h at 27 °C before fast filtration preparation of the samples for mass spectrometry analysis. Metabolites were analyzed by ionic-exchange chromatography coupled with tandem mass spectrometry (IC-MS/MS) using the method described by Bolten et al. Retention time on the column and multiple reaction monitoring (MRM) transition of each analyzed metabolite are known by the one skilled in the art.

The ¹³C mass isotopomer distribution of intracellular metabolites was determined from relevant isotopic clusters in the IC-MS/MS analysis, according to Kiefer et al. ¹³C mass isotopomer distribution measurements were performed using a triple quadrupole mass spectrometer (4000Qtrap, Applied Biosystems). To obtain ¹³C-labeling patterns (¹³C isotopologues), isotopic clusters were corrected for the natural abundance of isotopes other than 13C, using the in-house software IsoCor (available at MetaSys).

### OX- and OX- LUAD orthotopic mouse model

All procedures using animals were approved by the Animal Care and Use Ethics Committee of the University of Bordeaux. The NOD/LtSz-scid/IL-2Rychain^{null} (NSG) mice were produced and housed in the University Bordeaux Segalen animal facility, according to the rules and regulations governed and enforced by the Institutional Animal Care and Use Committee. The animal facility institutional agreement number is A33063916. Animals were included in protocols between 6 and 8 weeks old. Mice were monitored weekly for body weights and were also examined for aspect and behavior during the time-course of the experiments. No changes were noticed except otherwise indicated.

The orthotopic mouse models of OX+ or OX- human LUAD were obtained by using luciferase expressing clones of A549 or H460 lung adenocarcinoma cell lines, respectively. Groups of 5 mice were anesthetized with isoflurane and 1 × 10⁶ cells in a volume of 100µl were injected in the lung of 8-weeks NGS mice and imaged 20 min after injection. Bioluminescence imaging (BLI) was performed on a Bioimager following intraperitonal injection of 150 mg/kg of D-Luciferin (Interchim, Montluçon, France). After 10 min, mice were anesthetized by isoflurane and placed into a photon bioimager (BIOSPACE LAB, Paris, France) for about 20 min to acquire luminescence images. Signals were quantified with the M3Vision software (BIOSPACE LAB). Mice underwent BLI every two days for a period of 20 days. The mice were then euthanized, and the tumors were dissected and frozen or fixed.

### In vivo Trimetazidine treatment

Trimetazidine was purchased from Sigma and solubilized in saline solution. A dose of 20 mg/kg was injected intraperiteonally daily to the treated-group of mice.

### Statistics

All values are the mean ± SEM. The statistics were performed with GraphPad Prism 6 software using an unpaired Student's t test to compare two independent groups or pair for sequential measurements. One-way and two-way ANOVA were performed when comparing different groups

### Example 1: Definition and characterization of Non-Small Cell Lung Cancer (NSCLC) bioenergetics subsets with mitochondrial respiration

Mitochondrial respiration has been analyzed in intact surgical pieces obtained from nineteen patients with human non-small cell lung cancer (NSCLC) following surgery and dissection by the pathologist in two zones: the Ki67 positive-cancer region (PZ) and the cancer adjacent tissue (CAT or HT).

For each tumor and CAT, 5 to 10 pieces were prepared to consider intra-tissue heterogeneity.

Routine respiration that was sensitive to respiratory chain inhibition by cyanide was measured as describe above.

### Results

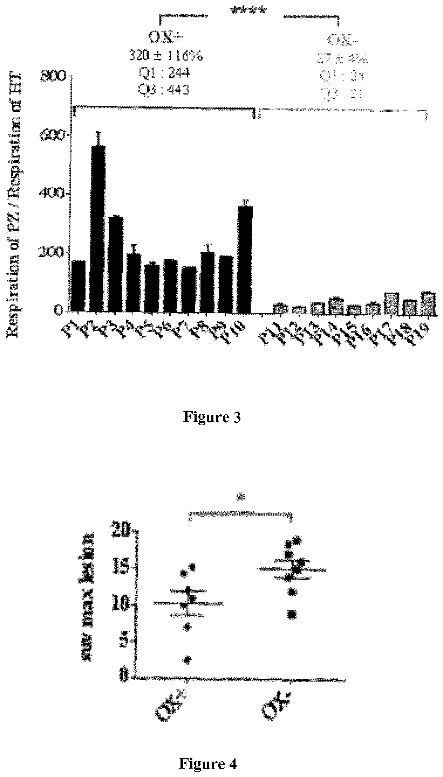

The absolute values of respiration as well as the respiration of the tumor normalized to that of the corresponding non-cancer tissue revealed two groups of tumors based on the respiratory rate as distinguished by the Anova analysis (Figures 2 and 3).

The group (N=10) with a high respiratory rate was denoted "OX+", and the group with low tumor respiration was denoted "OX-" (N=9). For each patient, the cancer adjacent tissue respiration systematically (p<0.05) opposed that of the tumor (Figure 2). Therefore, this functional analysis not only evidenced two types of lung tumors (OX+ and OX-) but also revealed the existence of a bioenergetic mispairing without precedent between the cancer area and the corresponding non-cancer tissue (OX+/HTₒₓ₋; OX-/HTₒₓ₊).

Calculation of the ratio between tumor and adjacent tissue respiration enabled a strong (p < 0.0001) segregation of the surgical pieces in two bioenergetic groups: OX+: high mitochondrial respiration and OX-: low mitochondrial respiration (Figure 3).

No significant difference in the respiratory control ratio (RCR) was found between the two groups (data not shown), indicating that the OX+ and OX- patients differed more by their capacity to perform respiration than their activation status of oxidative phosphorylation.

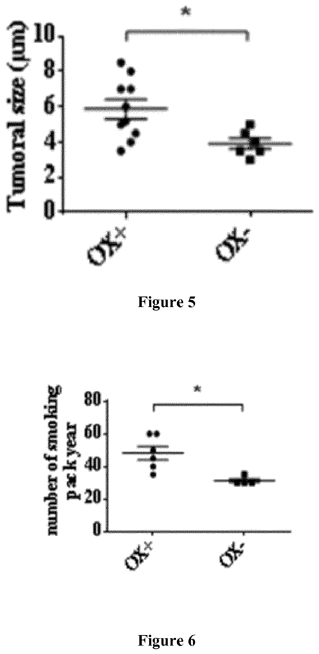

The clinical characteristics of the OX+ and OX- patients showed significant differences in the [¹⁸F]-fluorodeoxy-glucose (FDG) standardized uptake value (SUVmax) determined by positron emission tomography (PET), with lower values (p=0.0339) in the OX+ group (Figure 4). Tumor size (Figure 5) was also larger in the OX+ group (p=0.0197), whereas the tumor type, grade, c-stage, p-stage and KRAS or EGFR mutational status showed no significant difference between groups (data not shown). Lastly, the accumulated exposure to tobacco smoke (Figure 6) quantified by the number of cigarette pack-years (nPA) showed a significantly higher value in the OX+ patients (p=0.0129).

### Conclusions

Hence, this functional analysis evidenced two types of lung tumors: a group with high respiratory rate denoted: "OX+" (high mitochondrial respiration cancer) and a group with low respiratory rate denoted "OX-" (low mitochondrial respiration cancer).

Moreover, high mitochondrial respiration OX+ tumors are associated with a low mitochondrial respiration non-cancer tissue. In return low mitochondrial respiration OX- tumors are associated with high mitochondrial respiration non cancer tissue, supporting the concept of "bioenergetic mispairing" .

Furthermore, the analysis of the clinical data of the 19 patients who were enrolled in the functional study revealed differences between OX+ group and OX- group regarding 3 parameters obtained during the diagnosis: i) smoker status, (number of pack-years, nPa), ii) tumor size, and iii) incorporation of labeled glucose (FDG18) measured by PET scan.

These findings confirm the opposite correlation between [¹⁸F]-FDG PET status and mitochondrial respiratory activity. The OX + tumors thus exhibit strong mitochondrial respiration and a low SUV value.

### Example 2: Investigation of the molecular basis of the bioenergetic differences between the OX+ and OX- tumors

Comparative study using label-free proteomics was performed in order to investigate the molecular basis of the bioenergetics differences between the OX+ and OX- tumors.

This analysis was performed on six patients and identified 333 proteins that differed (p<0.05) between the OX+ and OX- tumors (Data not shown).

196 proteins were over-represented in the OX+ tumors (factor > 1.7), while 137 were repressed. A global analysis of the cellular functions (Ingenuity Pathways Analysis) impacted by the proteome remodelling between the OX+ and OX- tumors showed enrichment in phagosome maturation, clathrin- and caveolar-mediated endocytosis signalling and antioxidant response. A more detailed KEGG analysis of the metabolic enzymes overexpressed in the OX+ tumors revealed proteins involved in aldehyde detoxification, energy metabolism, carbohydrate metabolism and amino-acid metabolism (data not shown).

The differential proteomic signatures of the OX+ versus OX- tumors enabled the generation a molecular signature that was used to stratify the tumors on a larger panel of 522 human lung adenocarcinomas (RNA-Seq data) from The Cancer Genome Atlas (data not shown).

Hierarchical clustering revealed that the OX+ tumors (data not shown) represented 58% of the total of the lung adenocarcinomas (LUAD) population. The OX+ patients were more exposed to tobacco smoke than OX- (p=0.048), which confirmed the initial observations (Figure 6).

The OX+ tumors also contained more DNA copy number alterations and more point mutations than the OX- tumors, but there was no difference in the EGFR, ELM-ALK and KRAS mutational status. Unbiased GO and pathway enrichment analyses were performed using the top 500 genes upregulated in the OX+ and OX- tumors. The results indicated that the OX+ tumors were enriched for mRNAs involved in protein translation (68 genes) and energy metabolism (63 genes), including oxidative phosphorylation (37 genes) and the electron transport chain (37 genes) (data not shown).

### Example 3: Identification of the therapeutic target specific to OX+ lung tumors

To identify a potential therapeutic target that is specific to OX+ lung tumors, a comparative proteomic analysis of the tumor region with the corresponding CAT was performed using three OX+ patients (data not shown).

This study identified 154 proteins overexpressed in the tumor tissue with p<0.05, and a detailed analysis of the metabolic pathway suggested an oxidative shift, as revealed by a specific enrichment in proteins involved in fatty acid degradation and reduction in glycolytic enzymes (data not shown).

Moreover, catenin beta-1 (CTNNB1) was enriched in the OX+ tumors (fold change>8000 and p=3.8 x 10-3), and previous studies showed positive genetic regulation of HADHA by beta-catenin in hepatocytes 22.

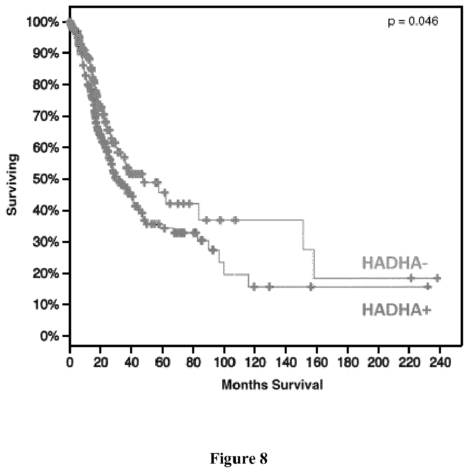

In silico analysis of HADHA expression in the TCGA cohort of human LUAD revealed a lower survival of the patients with high HADHA-tumor expression (Figure 8).

HADHB levels showed a positive correlation with that of HADHA in LUAD tumors (Data not shown).

A global analysis of the expression of the 115 genes involved in fatty acid metabolism, including transport, synthesis and degradation revealed two patient groups (Data not shown) characterized by low or high HADHA expression.

Venn diagrams (Data not shown) revealed that the OX+ subgroup (obtained from the heat map of extended data using the OX+ signature) showed a strong similarity with the HADHA+ group, which was enriched in FAO genes such as HADHA, HADHB, ECHS1 and ACSL3. The HADHA+ patients showed significant enrichment in genes of the respiratory chain involved in oxidative phosphorylation (i.e., ATP5O, ATP5H, ATP5L and ATP5C1). HADHA+ patient also showed a significant increase in the accumulated exposure to tobacco smoke (mean cigarette pack-years of 43.58 in HADHA+ vs 40.14 in HADHA-), enrichment in men gender (54.69% vs 37.93%) and reduction in EGFR mutation rate (31.31 % vs 51.53%), without difference in the KRAS mutation rate.

### Conclusions

The molecular basis of this phenomenon was analyzed in the OX+ tumors by comparative proteomics and revealed enrichment in FAO proteins and deprivation in glycolytic enzymes compared to the adjacent tissue.

These data further demonstrate that OX+ lung tumors overexpress MTP (HADHA and HADHB subunits).

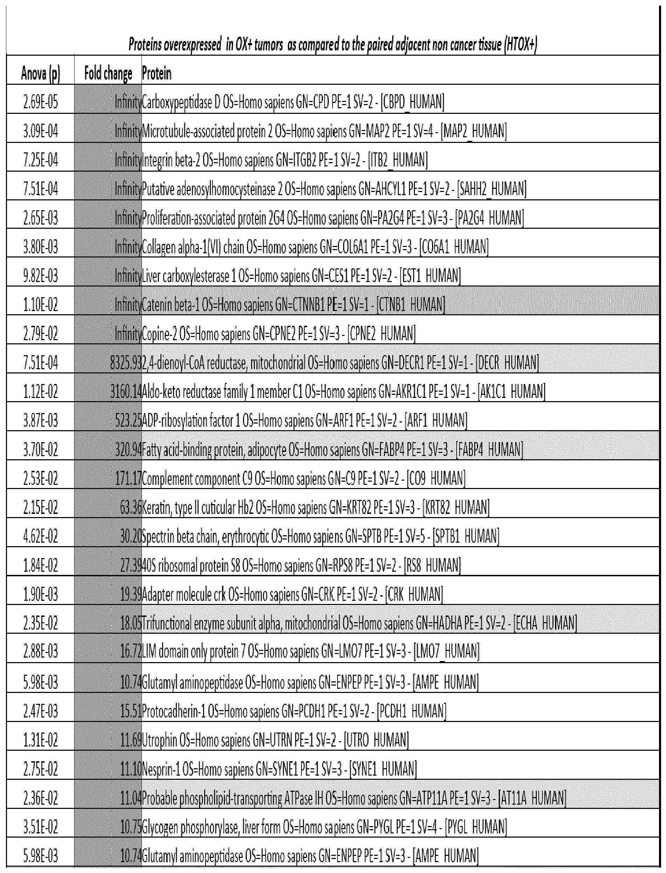

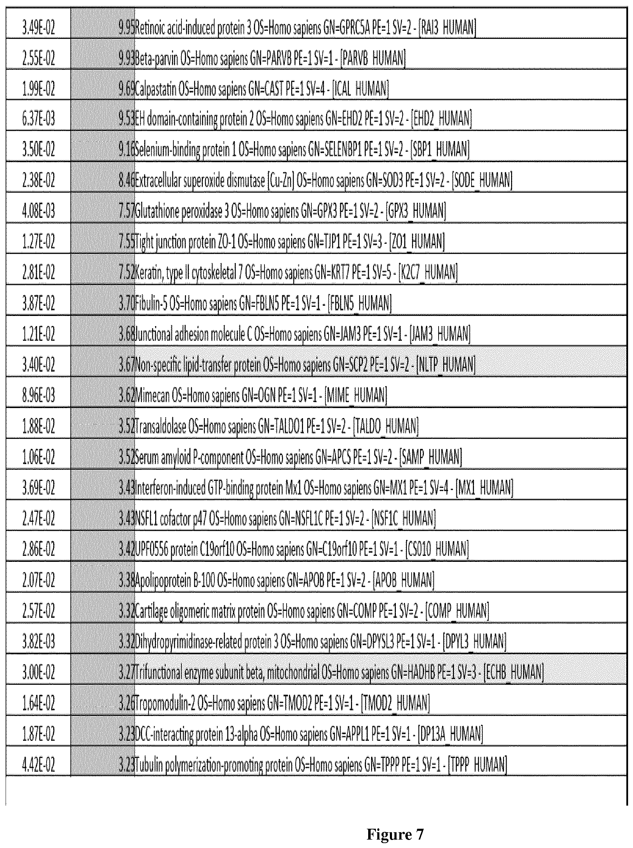

Indeed, as illustrated in Figure 7, protein involved in fatty acid oxidation are overexpressed in OX+ tumors compared to the paired adjacent non cancer tissue (HTOX+). The mitochondrial trifunctional protein (MTP) composed of alpha (HADHA) and beta (HADHB) subunits is also increased in OX+ tumors.

Hence, the mitochondrial trifunctional protein enzyme complex is a target of choice as this enzyme complex is the terminal step of fatty-acid oxidation (FAO) in the mitochondrion and the two subunits (HADHA and HADHB) are overexpressed in OX+ tumors.

Inhibition of HADHA or HADHB is thus relevant for inhibiting the growth of the tumor and thus for treating high mitochondrial respiration tumor (OX+), and in particular high mitochondrial respiration lung adenocarcinoma.

### Example 4: HADHA inhibition alters OX+ tumor growth in vitro and in vivo

### A549 human lung carcinoma cell lines

A549 and H460 human lung adenocarcinoma cell lines have contrasting bioenergetic profiles in good correspondence with high mitochondrial respiration OX+ and low mitochondrial respiration OX- lung adenocarcinoma, respectively.

First, it was observed that HADHA expression determined by Western Blot was higher in A549 cells (Figure 9) and that mitochondrial respiration was also higher in these cells (Figure 10).

Expression levels of four enzymes (ETFA, ETFB, ACADVL and Actin) involved in fatty-acid beta oxidation in the mitochondrion were also determined by Western Blot in the two cell lines. Significant increased expression levels are found in the A549 (OX+) cells (Figure 9).

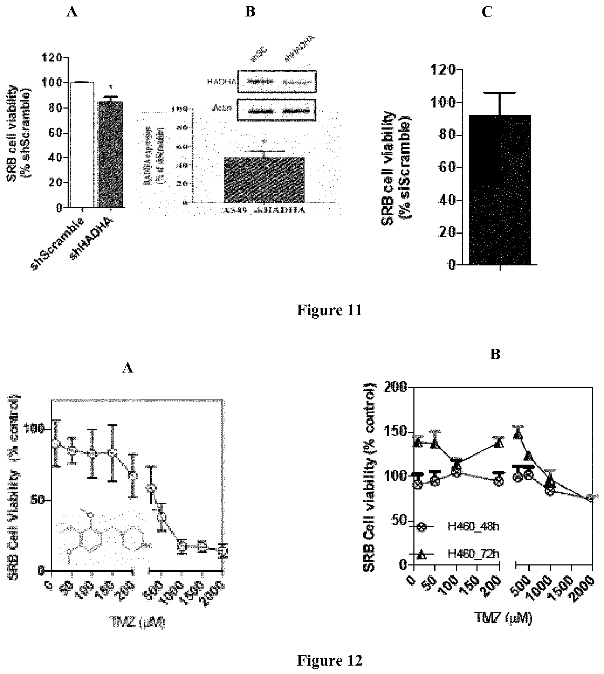

HADHA knockdown using siRNA and shRNA reduced the A549 cell number in both 2D and anchorage-independent growth conditions (Figures 11A et B) while no effect was observed on H460 cells (Figure 11 C).

Dose-dependent reduction of cell viability was also obtained using the MTP inhibitor Trimetazidine in A549 cells (Figure 12A) while no effect was seen on H460 cells (Figure 12B).

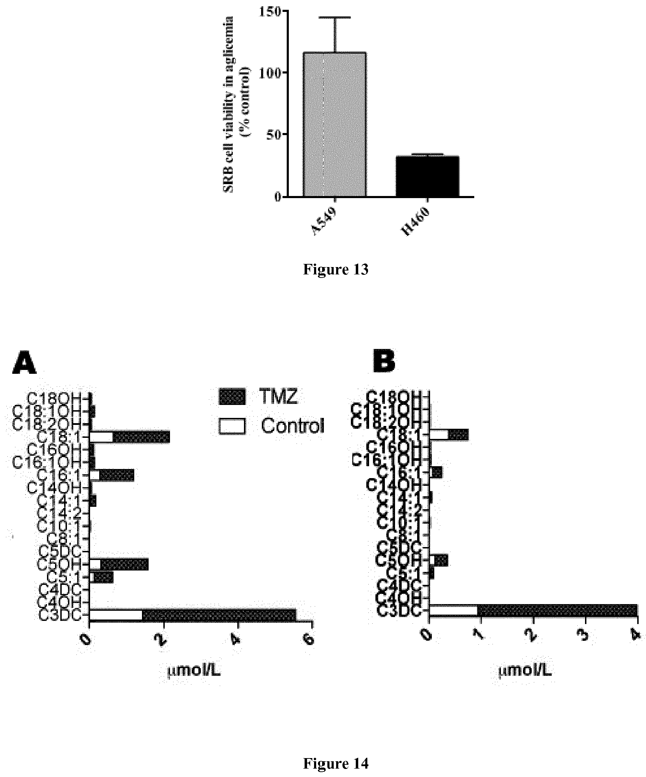

Conversely, H460 cells showed a stronger dependency for growth on glucose availability (Figure 13) and a higher sensitivity to the chemotherapeutic drugs 5-fluorouracile, doxorubicin and etoposide (data not shown).

The effect of HADHA knockdown on cell number was explained by a decrease in cell proliferation, as assessed by BrdU incorporation (data not shown).

### Orthotropic mouse model of human lung OX+ adenocarcinoma

To further investigate the strategy of targeting MTP in lung tumors, an orthotropic mouse model of human lung OX+ adenocarcinoma was developed, using A549 cells that stably expressed luciferase and a HADHA-targeted shRNA. The validity of this model was first verified by histology, which showed that anti-HLA-labeled A549 human cells formed orthotropic adenocarcinoma tumors in murine lungs (data not shown).

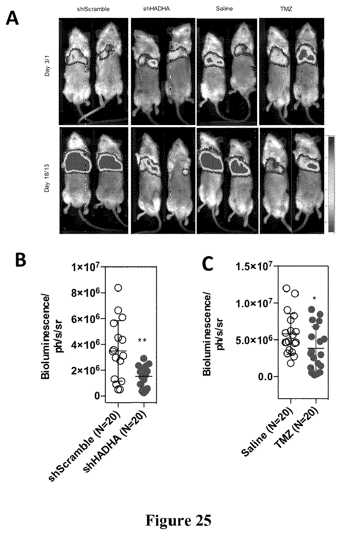

As illustrated in Figure 25, *in vivo* tumor size was determined using luminescence imaging of A549 cells expressing luciferase at day 3 and day 18 in four groups: shscramble, shHADHA, saline and TMZ (Figure 25A). Reduction of tumor size by shHADHA (Figure 25B) and by Trimetazidine 20mg/kg/day (Figure 25C) was demonstrated.

Hence, a significant reduction in tumor size was observed *in vivo* when HADHA expression was reduced using lentiviral shRNA in A549 cells, and similar results were obtained using the HADHA inhibitor trimetazidine (20 mg/kg, daily)

Of importance to the demonstration of bioenergetic-precision medicine, no effect of this drug was seen on the orthotropic mouse model of human lung OX- H460 adenocarcinoma.

### Conclusions

The present results demonstrate MTP inhibition using genetic or pharmacological means altered OX+ tumor growth in an orthotropic mouse model of OX+ lung adenocarcinoma.

It has also been demonstrated that trimetazidine, reduced tumor size in vivo, with no effect on OX-tumors. These findings provide proof-of-concept data for bioenergetic-precision medicine because OX+ A549 cancer cells were initially stratified by mitochondrial respiration and a lack of addiction to glucose.

### Example 5: Inhibition of respiratory chain complex I in A549 LUAD cells with Trimetazidine

Inhibition of MTP by trimetazidine in A549 cells triggered a significant reduction in the incorporation of [¹³C]-palmitate in the Krebs cycle as shown by a reduced content of [¹³C]citrate isotopomers after 2 hours as measured by the method hereinabove described. This inhibition of fatty acids degradation resulted in the accumulation of unprocessed fatty-acids inside A549 cells (Figure 14A) but not in H460 cells (Figure 14B).

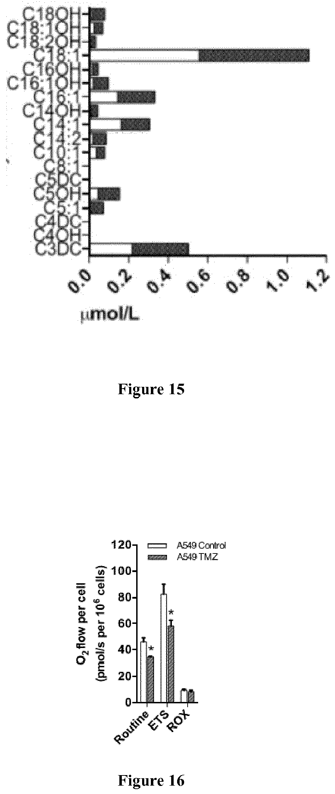

Fatty-acids accumulation was also found in the blood of OX+ mice treated with Trimetazidine (Figure 15). In particular, the level of hydroxylated fatty acids (C18-OH, C18-1OH, C18-2OH, C16OH, C14OH) was increased after Trimetazidine treatment.

In A549 cells, the large accumulation of sixteen carbon long fatty acids (C16:1 and C18:1) suggested that unprocessed free fatty-acids could also participate in the anti-cancer effect of Trimetazidine by inhibiting the respiratory chain.

Accordingly, it has been observed that Trimetazidine treatment of A549 cells inhibited mitochondrial respiration and altered the cellular ATP content as well as the NADH redox state (Figures 16, 17 and 18) compared to A549 cells not treated.

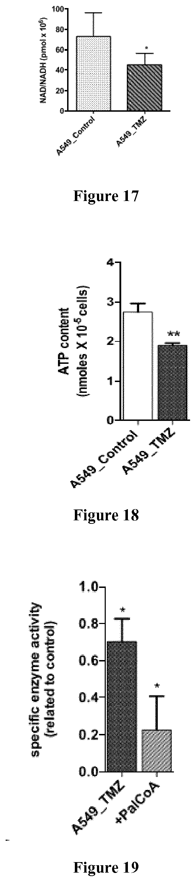

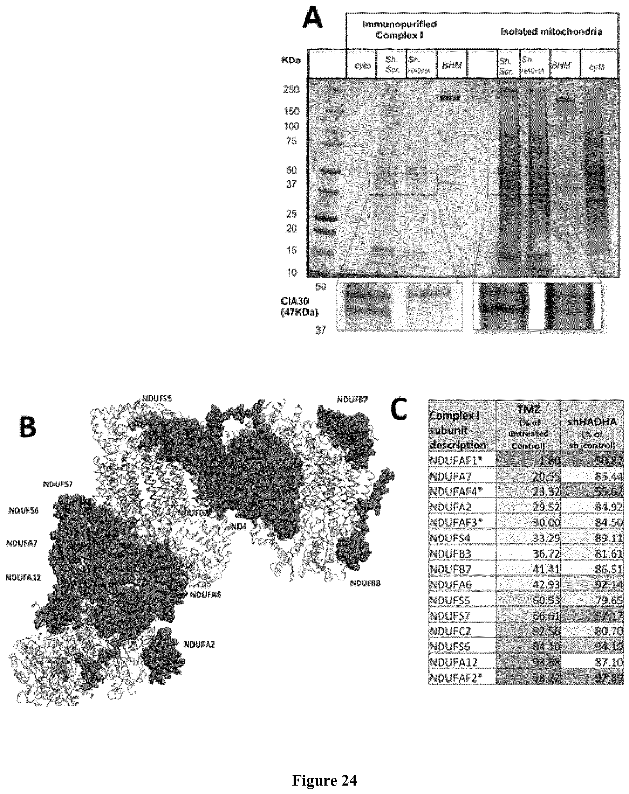

This effect of Trimetazidine on oxidative phosphorylation was explained by the inhibition of beta-oxidation combined with the inhibition of respiratory chain complex I. Complex I inhibition was also observed in A549 cells treated with palmitoyl-CoA (Figure 19) suggesting that unprocessed fatty-acids could participate to the bioenergetic impact of Trimetazidine.
The protein composition of immuno-purified complex I (Figure 24A) was analyzed. It has been observed a reduction in the several subunits, particularly in the range of 47Kda. Hence, a quantitative analysis of the proteome of A549 cells treated with TMZ or shHADHA (Figure 24B) was performed and revealed that in both models a significant reduction in the content of 15 Complex I subunits (Figure 24C). In particular, the Complex I intermediate-associated protein 30 (CIA30; NDUFAF1) was completely degraded after 4 hours of TMZ treatment and was decreased by 50% in shHADHA-expressing A549 cells. Previous work demonstrated that this subunit is essential for complex I assembly and activity.

### Conclusions

These findings provide proof-of-concept data for bioenergetic-precision medicine because OX+ A549 cancer cells were initially stratified by mitochondrial respiration and a lack of addiction to glucose. The mechanism of action of Trimetazidine could explain its strong impact on OX+ tumors *in vivo* since dual inhibition of oxidative phosphorylation was observed.

It has been demonstrated that trimetazidine and shHADHA alters the structure and subsequent catalytic activity of respiratory chain complex I. Molecular docking analysis and biochemical assays suggested that Coenzyme-A from palmitoyl-coA compete with the pantetheine moiety of PNS and triggers first the dissociation of the ACP subunit from the complex.

Consistent with the role of ACP in the biosynthesis of [FeS] clusters, It has been found that inhibition of HADHA also destabilized NDUFS2, the [FeS]-containing catalytic subunit of complex I. Additional complex I subunits were destabilized as determined by mass spectrometry: CIA30, NU4M, NU5M, NDUA7, NB5R1, NDUFA4, NDUA2, NDUF3, NDUS4, NDUB3, NDUB7, NDUA6, NDUS5, NDUS7, NDUC2, NDUS6 and NDUAC.

### Example 6: HADHA immunohistological scoring for OX+ patient stratification

Since the OX+ tumors are associated with a lower [¹⁸F]fluorodeoxy-glucose incorporation, the correlation between HADHA staining and the PET-scan SUVmax value was assessed by setting-up an HADHA immunohistology score (H-score).

First, little HADHA staining was observed in normal human lung adjacent to the tumors, whereas a significantly higher staining intensity was observed in the tumor region (data not shown).

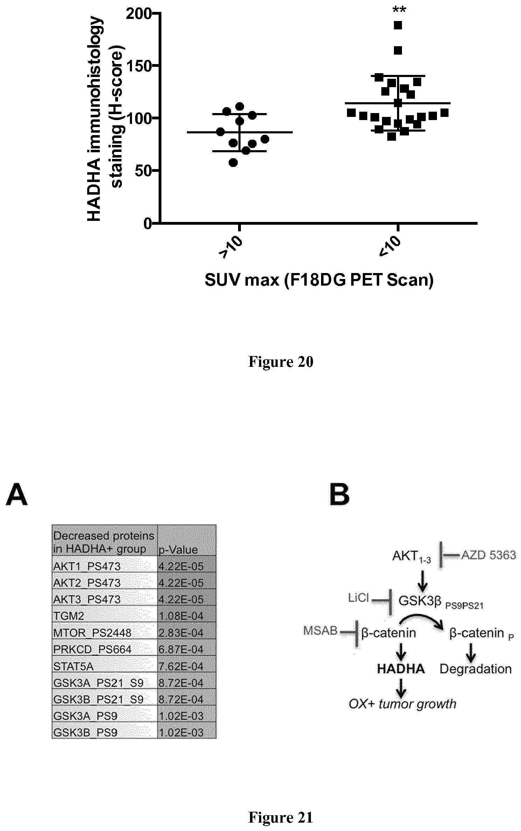

Moreover, HADHA staining showed higher H-score in patients with low PET-scan SUVmax values (Figure 20). These findings suggested that the SUV-max and HADHA immunostaining could be used to stratify HADHA+ and HADHA- patients.

Previous work showed that HADHA expression is controlled by beta-catenin and it has been observed an increased content of beta-catenin in OX+ tumors suggesting that a similar regulation could occur (data not shown).

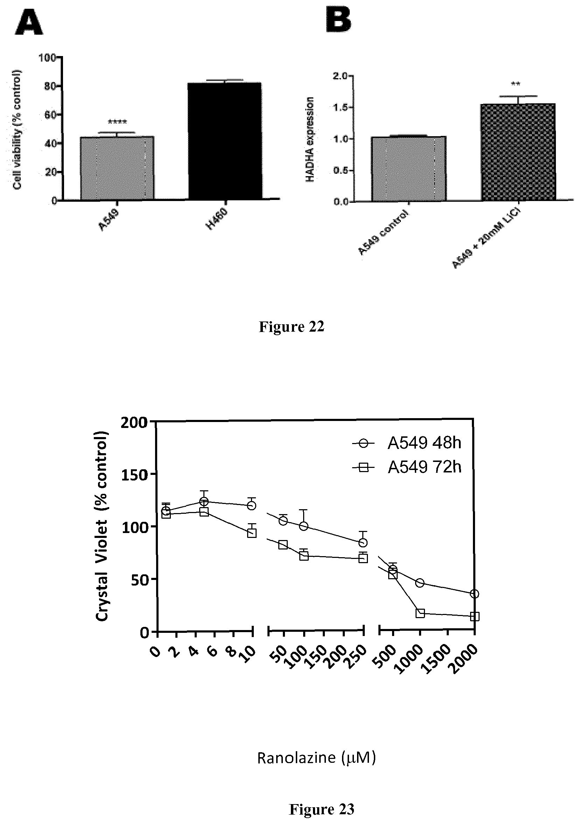

Moreover, an enrichment analysis performed in silico on HADHA-overexpressing tumors from the TCGA lung adenocarcinoma (LUAD) cohort showed a reduction in the active (phosphorylated) forms of AKT and GSK3B peptides (Figure 21A). Therefore, it is hypothesized that AKT and GSK3beta could act as negative regulators of HADHA expression given their known capacity to inhibit beta-catenin signalling (Figure 21B). Accordingly, the inhibition of GSK3B using lithium-chloride, a beta-catenin activator, stimulated the expression of HADHA in A549 cells (Figure 22B) and the direct inhibition of beta-catenin by MSAB triggered the opposite effect (Figure 22A).

### Example 7: Ranolazine inhibits A549 cells viability

A549 cells have been treated with Ranolazine and cell viability has been measured. A dose-dependent loss in A549 cell viability is observed upon Ranolazine treatment (Figure 23).

### Conclusions

Hence, metabolic reprogramming is a common hallmark of human tumors. Using high-resolution respirometry, two human lung adenocarcinoma subgroups have been identified: high (OX+) mitochondrial respiration and low (OX-) mitochondrial respiration. The OX+ tumors poorly incorporated [¹⁸F]fluorodeoxy-glucose and showed increased expression of the mitochondrial trifunctional protein (HADHA and HADHB subunits) compared to the adjacent tissue.

Genetic inhibition of HADHA altered OX+ tumor growth *in vivo.* Trimetazidine, an inhibitor of MTP, also reduced tumor growth and induced the accumulation of unprocessed fatty acids with consecutive destabilization of respiratory chain complex I catalytic subunit NDUFS2, leading to a cellular redox and energy crisis. HADHA expression in tumors varied in negative correlation with [¹⁸F]fluorodeoxy-glucose incorporation, and patients with high HADHA-expressing tumors had lower survival rates. These findings provide proof-of-concept data for preclinical precision bioenergetic medicine in lung tumors and for the use of HADHA inhibitors for the treatment of high respiratory mitochondrial cancers.

## Claims

1. MTP inhibitor for use in the treatment of a high mitochondrial respiration (OX+) cancer.

2. MTP inhibitor for use according to claim 1, wherein the MTP inhibitor is a HADHA inhibitor or a HADHB inhibitor.

3. MTP inhibitor for use according to claims 1 or 2, wherein the MTP inhibitor is Trimetazidine or Ranolazine or 4-pentenoic acid.

4. MTP inhibitor for use according to anyone of claims 1 to 3, wherein the MTP inhibitor is Trimetazidine.

5. MTP for use inhibitor according to anyone of claims 1 to 4, wherein said high mitochondrial respiration (OX+) cancer is chosen among prostate cancer, lymphomas, leukemia, melanomas, lung cancers, ovarian tumors, breast tumors, kidney cancers and hepatoma.

6. MTP inhibitor for use according to anyone of claims 1 to 5, wherein said high mitochondrial reparation (OX+) cancer is lung cancer, preferably non-small cell lung cancer.

7. MTP inhibitor for use according to anyone of claims 1 to 6, wherein the high mitochondrial respiration (OX+) cancer is a lung adenocarcinoma.

8. MTP inhibitor for use according to claim 1 or 2, wherein the high mitochondrial respiration (OX+) cancer is further a high fatty-acid oxidation (FAO+) cancer.

9. MTP inhibitor for use according to claim 8, wherein the high mitochondrial respiration (OX+) and high fatty-acid oxidation (FAO+) cancer is chosen among lung cancer and lymphoma.

10. MTP inhibitor for use according to claim 8 or 9, wherein the high mitochondrial respiration (OX+) and high fatty-acid oxidation (FAO+) cancer is lung adenocarcinoma.

11. MTP inhibitor for use according to anyone of claims 1 to 10, wherein said HADHA inhibitor is used in combination with any other therapeutic strategy chosen among surgery, external radiotherapy and chemotherapy.

12. MTP inhibitor for use according to claim 11, wherein said HADHA inhibitor is used in combination with a beta-catenin inhibitor.

13. An in vitro method of determining the prognosis of a subject suffering from a cancer, comprising the step of measuring the expression of MTP in tumors of cancers chosen among prostate cancer, lymphomas, leukemia, melanomas, ovarian tumors, breast tumors, kidney cancer, hepatomas and lung cancers.

14. An in vitro method of determining the prognosis of a subject suffering from a cancer according to claim 13, wherein the cancer is a lung adenocarcinoma.


SEQUENCE LISTING

<110>  INSERM ; Université de Bordeaux

<120>  Mitochondrial trifunctional protein (MTP) inhibitors for use in
       the treatment of high mitochondrial respiration (OX+) cancers

<130>  BEP160917

<160>  3

<170>  PatentIn version 3.5

<210>  1
<211>  20
<212>  DNA
<213>  primers

<400>  1
actaaaacct ccagaggaac                                                   20


<210>  2
<211>  20
<212>  DNA
<213>  primer

<400>  2
gtcaattttt ccaccaatcc                                                   20


<210>  3
<211>  29
<212>  DNA
<213>  shHADHA

<400>  3
aagcctattg tggctgccat caatggatc                                         29